# EUROPEAN PATENT SPECIFICATION

(11) **EP 2 612 692 B1**
(45) Date of publication and mention of the grant of the patent: **11.09.2019**
(21) Application number: 11821510.2
(22) Date of filing: 03.08.2011
(51) Int. Cl.: A61N 5/10, G21K 1/00, G21K 5/00, G21K 5/02, H05H 3/06, H05H 6/00, H05H 13/00, G21K 5/04, G21K 5/08, G21K 5/10

(54) **NEUTRON BEAM IRRADIATION SYSTEM**
NEUTRONENSTRAHLUNGSSYSTEM
SYSTÈME D'IRRADIATION À FAISCEAU DE NEUTRONS

(30) Priority: 01.09.2010 JP 2010195835
(43) Date of publication of application: 10.07.2013
(73) Proprietor: Sumitomo Heavy Industries, Ltd., Tokyo 141-6025 (JP)
(72) Inventor: MITSUMOTO Toshinori, Nishitokyo-shi Tokyo 188-8585 (JP)
(74) Representative: Carstens, Dirk Wilhelm
(86) International application number: PCT/JP2011/067795
(87) International publication number: WO 2012/029492

(56) References cited:
- JP-A- 58 103 699
- JP-A- 2007 089 928
- JP-A- 2008 022 920
- JP-A- 2009 189 725
- JP-A- 2009 204 428

## Description

### Technical Field

The present invention relates to a neutron beam irradiation system.

### Background Art

As one radiation therapy in cancer therapy or the like, there is a boron-neutron capture therapy (BNCT: BoronNCT) in which cancer therapy is performed by irradiation of a neutron beam. In the past, a neutron beam irradiation system (a BNCT system) for performing the boron-neutron capture therapy has been developed, and for example, in JP 2009-189725 A, there is disclosed a neutron beam irradiation system which is provided with a neutron beam generating section (a target) which is irradiated with a charged particle beam, thereby generating a neutron beam, a neutron beam converging section which converges the neutron beam that is irradiated, and a placement table on which an irradiated body such as a patient is placed. In the neutron beam irradiation system stated in JP 2009-189725 A, the irradiated body on the placement table is irradiated with the neutron beam generated in the neutron beam generating section, through the neutron beam converging section. Further, in the neutron beam irradiation system, a configuration is made such that the neutron beam generating section is accommodated by an accommodation section which includes a moderator.

### Summary of Invention

### Technical Problem

Here, in a case where the irradiated body is irradiated with the neutron beam by using the neutron beam irradiation system described above, for example, immediately after the irradiation of the neutron beam, the neutron beam generating section, the accommodation section, and the neutron beam converging section are often relatively radioactivated, and thus in order for a medical doctor or the like to approach the placement table, it is necessary to wait for such radioactivation to be attenuated. Therefore, in the neutron beam irradiation system described above, there is a concern that work efficiency may become low.

Therefore, the present invention has an object of providing a neutron beam irradiation system in which work efficiency can be enhanced.

### Solution to Problem

In order to solve the above-described problem, according to the present invention, there is provided a neutron beam irradiation system as set forth in claim 1. Preferred embodiments of the present invention may be gathered from the dependent claims.

In the neutron beam irradiation system, for example, also immediately after the irradiation of the neutron beam, by separating the placement table from the radioactivated neutron beam generating section, accommodation section, and neutron beam converging section, a medical doctor or the like can approach the placement table without being adversely affected by radioactivation, and thus the need to wait for the radioactivation to be attenuated can be reduced. Therefore, it becomes possible to enhance work efficiency.

The neutron beam irradiation system includes a first radiation shielding door that is disposed between the neutron beam generating section and the placement table in a state where the neutron beam generating section and the placement table are separated from each other with respect to a positional relationship at the time of the irradiation of the neutron beam. In this way, radiation can be effectively shielded between the neutron beam generating section and the placement table.

Further, it is preferable that the movement means is adapted to move the neutron beam converging section relative to the neutron beam generating section so as to approach and be separated from the neutron beam generating section. In this case, it is possible to perform alignment of the irradiated body and the neutron beam converging section in a state where the irradiated body on the placement table is separated from, for example, the neutron beam generating section.

At this time, it is preferable that the neutron beam irradiation system further include a second radiation shielding door that is disposed between the neutron beam generating section and the neutron beam converging section in a state where the neutron beam generating section and the neutron beam converging section are separated from each other with respect to a positional relationship at the time of the irradiation of the neutron beam. In this way, radiation can be effectively shielded between the neutron beam generating section and the placement table.

Further, as a configuration of suitably exhibiting the above-described operation and effects, specifically, a configuration can be given in which the relative movement means includes a rail.

Further, it is preferable that the neutron beam irradiation system further include a radiation shielding wall that accommodates the accommodation section so as to be embedded therein. In this case, unintended radiation from the neutron beam generating section can be effectively shielded such that the radiation does not reach the irradiated body side.

Further, it is preferable that the movement means is adapted to move the accommodation section relative to the neutron beam generating section so as to approach and be separated from the neutron beam generating section. In this case, at the time of maintenance, by moving the accommodation section relative to the neutron beam generating section by the movement means so as to be separated from the neutron beam generating section, it becomes possible to easily access the neutron beam generating section and the accommodation section, and thus maintenance, replacement, or the like of the accommodation section can be conveniently performed without, for example, removal to a peripheral site or removing peripheral equipment. Therefore, it becomes possible to easily perform maintenance of the neutron beam irradiation system.

At this time, it is preferable that the neutron beam generating section be fixed to an installation floor and the movement means is adapted to move the accommodation section relative to the neutron beam generating section by moving the accommodation section with respect to the installation floor. In this case, the beam length of the charged particle beam which is irradiated to the neutron beam generating section is reduced, and thus it becomes easy to attain a reduction in the size of the system.

### Advantageous Effects of Invention

According to the invention, it becomes possible to enhance work efficiency.

### Brief Description of Drawings

FIG. 1 is a diagram showing the configuration of a neutron beam irradiation system related to an embodiment.
FIG. 2 is a cross-sectional perspective view showing a neutron beam generation unit and a treatment table in the neutron beam irradiation system of FIG. 1.
FIG. 3 (a) is a diagram showing the time of maintenance of the neutron beam irradiation system of FIG. 1 and FIG. 3(b) is a diagram showing a situation succeeding that of FIG. 3(a).
FIG. 4 (a) is a diagram for describing an operation of the neutron beam irradiation system of FIG. 1 and FIG. 4(b) is a diagram showing a situation succeeding that of FIG. 4(a).
FIG. 5 (a) is a diagram showing a situation succeeding that of FIG. 4(b) and FIG. 5(b) is a diagram showing a situation succeeding that of FIG. 5(a).

### Description of Embodiments

Hereinafter, a preferred embodiment of the invention will be described in detail with reference to the drawings. In addition, in the following description, the same or equivalent elements are denoted by the same reference numerals and overlapping description is omitted. Further, the terms "upstream" and "downstream" respectively mean upstream (a cyclotron side) and downstream (an irradiated body side) of a charged particle beam and a neutron beam which are emitted.

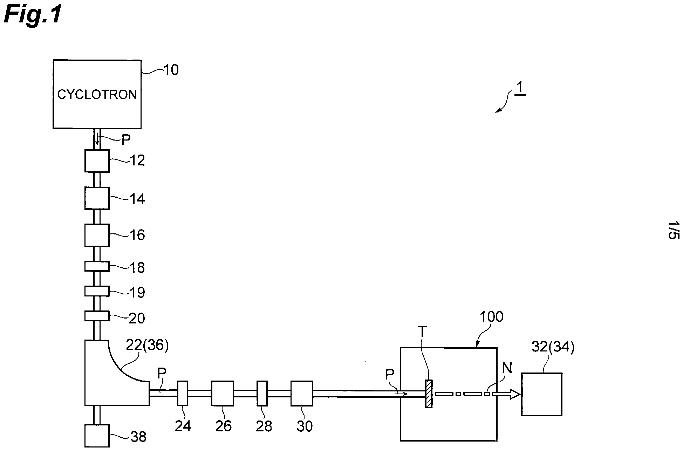

FIG. 1 is a diagram showing the configuration of a neutron beam irradiation system related to an embodiment. As shown in FIG. 1, a neutron beam irradiation system 1 is an apparatus that is used in order to perform cancer therapy or the like using, for example, neutron capture therapy and irradiates an irradiated body 34 such as a patient with a neutron beam N.

The neutron beam irradiation system 1 is provided with a cyclotron 10, and the cyclotron 10 accelerates charged particles such as protons, thereby producing a proton beam as a charged particle beam P. The cyclotron 10 here has the ability to produce, for example, a 60 kW (= 30 MeV×2 mA) charged particle beam P having a beam radius of 40 mm.

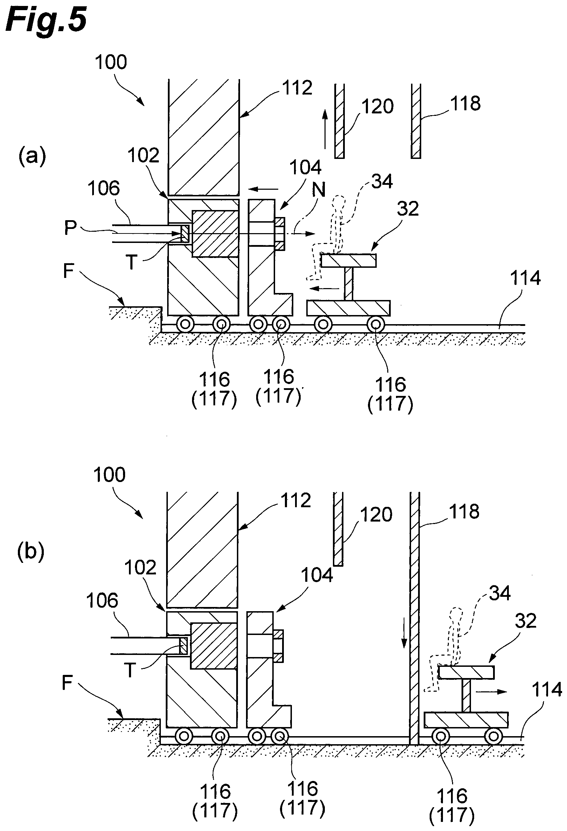

The charged particle beam P taken out of the cyclotron 10 sequentially passes through a horizontal type steering 12, a 4-direction slit 14, a horizontal and vertical type steering 16, quadrupole electromagnets 18, 19, and 20, a 90-degree deflection electromagnet 22, a quadrupole electromagnet 24, a horizontal and vertical type steering 26, a quadrupole electromagnet 28, and a 4-direction slit 30 and is led to a neutron beam generation unit 100. The charged particle beam P is irradiated to a target (a neutron beam generating section) T in the neutron beam generation unit 100, whereby the neutron beam N is generated. Then, the neutron beam N is irradiated to the irradiated body 34 on a treatment table (a placement table) 32 (refer to FIG. 5).

The horizontal type steering 12 and the horizontal and vertical type steering 16 and 26 are for performing beam axis adjustment of the charged particle beam P by using, for example, an electromagnet. Similarly, the quadrupole electromagnets 18, 19, 20, 24, and 28 are for suppressing beam divergence of the charged particle beam P by using, for example, an electromagnet. The 4-direction slits 14 and 30 are for detecting the beam position of the charged particle beam P by interrupting edges of the beam.

The 90-degree deflection electromagnet 22 is for performing 90-degree deflection of a traveling direction of the charged particle beam P. In addition, a switching section 36 is provided in the 90-degree deflection electromagnet 22, and by the switching section 36, it becomes possible to make the charged particle beam P deviate from a regular trajectory, thereby leading to a beam dump 38. The beam dump 38 performs output confirmation of the charged particle beam P before therapy or the like.

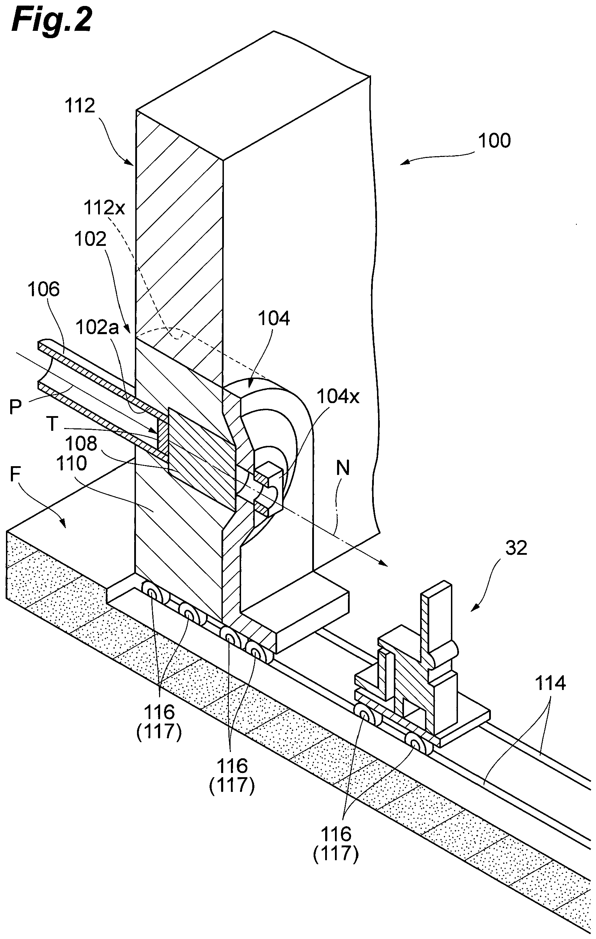

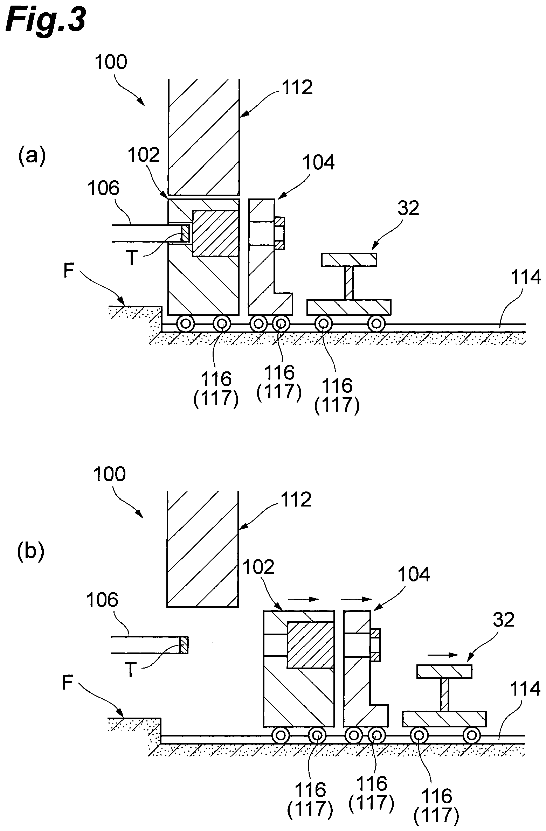

FIG. 2 is a cross-sectional perspective view showing the neutron beam generation unit and the treatment table in the neutron beam irradiation system of FIG. 1, FIG. 3 is a flow diagram showing the time of maintenance of the neutron beam irradiation system of FIG. 1, and FIGS. 4 and 5 are flow diagrams for describing an operation of the neutron beam irradiation system of FIG. 1. As shown in FIG. 2, the neutron beam generation unit 100 includes the target T which is irradiated with the charged particle beam P, thereby generating the neutron beam N, an accommodation section 102 that accommodates the target T, and a converging section (a neutron beam converging section) 104 that converges the neutron beam N.

The target T is formed of, for example, beryllium (Be) and has a disc shape having a diameter of 160 mm. The target T is disposed at a downstream end portion of a beam duct 106 that passes the charged particle beam P therethrough. The beam duct 106 is fixed to an installation floor F on which the neutron beam generation unit 100 is installed, thereby being configured so as to be unable to relatively move with respect to the installation floor F. That is, the target T is also fixed so as to be unable to relatively move with respect to the installation floor F.

The accommodation section 102 is configured to include a moderator 108 and a shielding material 110. The moderator 108 is for decelerating the neutron beam N generated in the target T and has, for example, a laminated structure which is composed of a plurality of different materials. The shielding material 110 is provided so as to cover the moderator 108 and reflects and absorbs the neutron beam N and radiated rays (hereinafter simply referred to as "radiation") such as gamma rays produced with the generation of the neutron beam N, thereby shielding the neutron beam N and the radiation so as not to be released to the outside.

In the accommodation section 102, as described above, the target T is accommodated in the inside. Specifically, an opening 102a that is opened to the upstream side is formed in the accommodation section 102, and a downstream end portion of the beam duct 106 is disposed in the opening 102a, and thus the target T is accommodated in the accommodation section 102 so as to be surrounded.

The converging section 104 is for converging the neutron beam N that is irradiated, thereby adjusting an irradiation range of the neutron beam N, and is disposed further to the downstream side than the accommodation section 102 in a beam path of the neutron beam N. The converging section 104 is configured to include a collimator 104x. The treatment table 32 is for placing the irradiated body 34 (refer to FIG. 5) thereon and is disposed further to the downstream side than the converging section 104 in the beam path of the neutron beam N.

Here, the accommodation section 102, the converging section 104, and the treatment table 32 in this embodiment are respectively configured so as to be movable along a plurality of rails 114 laid on the installation floor F. That is, sliding sections 116 such as wheels or sliders provided at a lower end portion of each of the accommodation section 102, the converging section 104, and the treatment table 32 are engaged with the rails 114 extending along an irradiation direction (a beam axis direction) of the neutron beam N. Then, the sliding sections 116 are driven by a driving section 117 such as a motor, for example, and thus each of the accommodation section 102, the converging section 104, and the treatment table 32 is made to be able to move on the rails 114. In this way, the accommodation section 102, the converging section 104, and the treatment table 32 are made to be able to independently move on the installation floor F along the rails 114.

As a result, the accommodation section 102, the converging section 104, and the treatment table 32 can move relative to the target T so as to approach and be separated from the target T along the irradiation direction of the neutron beam N. That is, the treatment table 32 is made to be able to approach and be separated from the target T, the accommodation section 102, and the converging section 104 along the irradiation direction of the neutron beam N. Further, here, since the accommodation section 102, the converging section 104, and the treatment table 32 are independently movable, as described above, the accommodation section 102, the converging section 104, and the treatment table 32 are made to be movable relative to each other so as to approach each other and be separated from each other along the irradiation direction of the neutron beam N.

Therefore, at the time of maintenance of the neutron beam irradiation system 1, as shown in FIGS. 3(a) and 3(b), by moving the accommodation section 102 with respect to the target T along the rails 114 so as to be separated from the target T, it becomes possible to create a state where the target T (the beam duct 106) is not accommodated in the accommodation section 102 and then easily access the target T and the accommodation section 102. Therefore, maintenance, replacement, or the like of the accommodation section 102 can be conveniently performed without, for example, removal to a peripheral site or removing peripheral equipment, and thus it becomes possible to easily perform the maintenance of the neutron beam irradiation system 1.

In particular, in this embodiment, as described above, the converging section 104 and the treatment table 32 are also made to be movable with respect to the target T. Therefore, at the time of the maintenance, by moving the converging section 104 and the treatment table 32 relative to the target T such that the converging section 104 and the treatment table 32 are also separated from the target T, it becomes possible to more easily access the target T and the accommodation section 102, and thus such maintenance can be more easily performed.

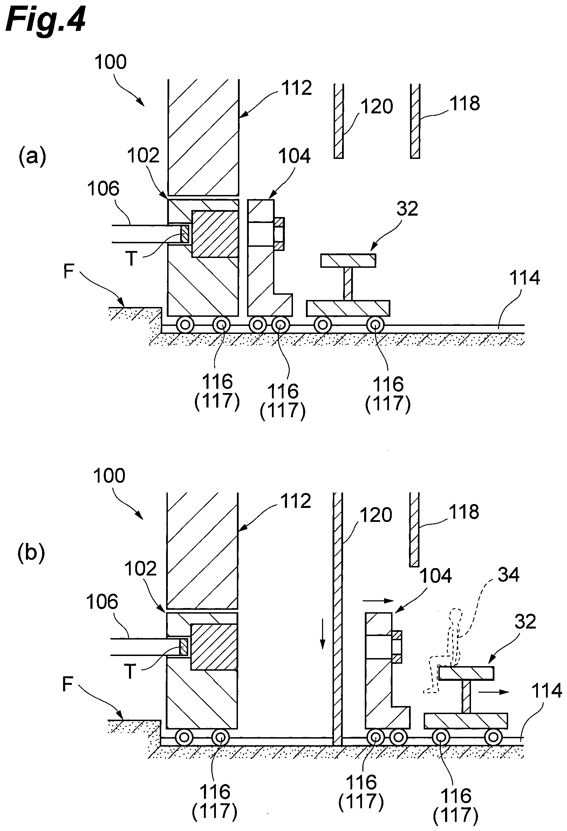

Returning to FIG. 2, the neutron beam irradiation system 1 is provided with a radiation shielding wall 112 and first and second radiation shielding doors 118 and 120 (refer to FIG. 4) as members for shielding radiation. The radiation shielding wall 112 accommodates the accommodation section 102 so as to be embedded in the inside thereof. Specifically, the entirety of the accommodation section 102 is disposed and accommodated so as to be fitted into a through-hole 112x having a shape in accordance with the outer shape of the accommodation section 102 in the radiation shielding wall 112.

As shown in FIG. 5(b), the first radiation shielding door 118 is opened and closed so as to be disposed between the target T and the treatment table 32 in a state where the target T and the treatment table 32 are separated from each other with respect to a positional relationship (refer to FIG. 4(a)) at the time of the irradiation of the neutron beam N. Further, as shown in FIG. 4(b), the second radiation shielding door 120 is opened and closed so as to be disposed between the target T and the converging section 104 in a state where the target T and the converging section 104 are separated from each other with respect to a positional relationship (refer to FIG. 4(a)) at the time of the irradiation of the neutron beam N. The first and second radiation shielding doors 118 and 120 here are formed of lead (Pb) or the like.

Next, an operation of the neutron beam irradiation system 1 will be described. In the neutron beam irradiation system 1 in the initial state shown in, for example, FIG. 4(a), first, as shown in FIG. 4(b), the converging section 104 and the treatment table 32 are relatively moved along the rails 114 so as to be separated from the target T by a distance greater than or equal to a predetermined distance. Then, the second radiation shielding door 120 is closed and also the irradiated body 34 is placed on the treatment table 32, and thereafter, alignment of the converging section 104 and the irradiated body 34 is performed. In addition, the predetermined distance described above is set to be such a distance that the effect of radiation around, for example, the target T becomes very small (the same also applies to the following predetermined distance) .

Subsequently, as shown in FIG. 5(a), the second radiation shielding door 120 is opened and also the converging section 104 and the treatment table 32 are relatively moved along the rails 114 so as to approach the target T while the alignment state is maintained. In this way, the target T, the converging section 104, and the treatment table 32 have a positional relationship in which the irradiated body 34 is properly irradiated with the neutron beam N. Then, the neutron beam N is generated by irradiating the target T with the charged particle beam P by operating the cyclotron 10 (refer to FIG. 1), and the irradiated body 34 is irradiated with the neutron beam N through the converging section 104.

Subsequently, after the irradiation of the neutron beam N, as shown in FIG. 5(b), the treatment table 32 is relatively moved with respect to the target T, the accommodation section 102, and the converging section 104 along the rails 114 so as to be separated by a distance greater than or equal to the predetermined distance. Then, the first radiation shielding door 118 is closed. By the above, the operation of the neutron beam irradiation system 1 is finished.

As described above, in the neutron beam irradiation system 1 related to this embodiment, after the irradiation of the neutron beam N, the treatment table 32 is separated from the target T, the accommodation section 102, and the converging section 104 by a distance greater than or equal to the predetermined distance. Therefore, after the irradiation of the neutron beam N, a medical doctor, a worker, or the like (hereinafter referred to as a "medical doctor or the like") can immediately approach under the treatment table 32 without being adversely affected by radioactivation, and thus the need to wait for the radioactivation to be attenuated can be reduced. As a result, according to this embodiment, it becomes possible to enhance the work efficiency (the treatment efficiency) of the neutron beam irradiation system 1. Further, in this manner, by moving the treatment table 32 away after the irradiation, it is possible to suppress the effect of the residual radiation around the target T on the irradiated body 34.

Then, after the irradiation of the neutron beam N, since the first radiation shielding door 118 is closed and disposed between the target T and the treatment table 32 (refer to FIG. 5(b)), the radiation is effectively shielded between the target T and the treatment table 32, and thus the effect of the residual radiation around the target T on the irradiated body 34 can be further suppressed.

Further, in this embodiment, as described above, the converging section 104 can also move relative to the target T so as to approach and be separated from the target T, and alignment of the irradiated body 34 (the treatment table 32) and the converging section 104 is performed in a state where the irradiated body 34 is separated from the target T before the irradiation of the neutron beam N. Therefore, at the time of such alignment, the effect of the residual radiation around the target T on the irradiated body 34 can be suppressed.

Then, before the irradiation of the neutron beam N, since the second radiation shielding door 120 is closed and disposed between the target T and the converging section 104 (refer to FIG. 4(b)), the radiation is effectively shielded between the target T and the converging section 104, and thus the effect of the residual radiation around the target T on the irradiated body 34 can be further suppressed.

Further, in this embodiment, as described above, the accommodation section 102 is accommodated by the radiation shielding wall 112 so as to be embedded therein. Therefore, unintended radiation from the target T can be effectively shielded such that the radiation does not reach the irradiated body 34 side. It becomes possible to suppress the radiation released outside the accommodation section 102 toward the downstream side from, for example, the opening 102a being turned and advancing to the upstream side and reaching the irradiated body 34.

Further, in this embodiment, as described above, as movement means that moves the accommodation section 102 relative to the target T so as to approach and be separated from the target T, the rail 114, the sliding section 116, and the driving section 117 are provided. Therefore, at the time of maintenance, by moving the accommodation section 102 relative to the target T so as to be separated from the target T, it is possible to easily access the target T and the accommodation section 102. As a result, easier maintenance of the neutron beam irradiation system 1 becomes realizable, and thus shortening of working hours becomes possible. In addition, in a case where the medical doctor or the like accesses, for example, the accommodation section 102, since the accommodation section 102 is separated from the target T, the effect of the residual radiation around the target T on the medical doctor or the like can be suppressed.

Further, in this embodiment, as described above, the target T is fixed to the installation floor F, and the accommodation section 102 is moved relative to the target T by moving the accommodation section 102 with respect to the installation floor F. Therefore, restriction in terms of disposition of, for example, the cyclotron 10 or restriction at the time of maintenance can be reduced and it becomes easy to reduce the beam length of the charged particle beam P which is irradiated to the target T. As a result, it becomes possible to easily attain a reduction in the size of the neutron beam irradiation system 1.

In the above, the rail 114, the sliding section 116, and the driving section 117 constitute the movement means.

The preferred embodiment has been described above. However, the invention is not limited to the embodiment described above and may also be modified within the scope of the appended claims.

For example, in the above-described embodiment, the target T is fixed and the accommodation section 102 is moved with respect to the target T. However, a configuration is also possible in which the accommodation section 102 is fixed and the target T is moved with respect to the accommodation section 102. Similarly, a configuration is also possible in which the converging section 104 is fixed and the target T is moved with respect to the converging section 104, and a configuration is also possible in which the treatment table 32 is fixed and the target T is moved with respect to the treatment table 32.

Further, in the above-described embodiment, the movement means is configured to include the rail 114, the sliding section 116, and the driving section 117. However, there is no limitation thereto, and various movement means can be adopted.

Further, in the above-described embodiment, the first and second radiation shielding doors 118 and 120 are provided. However, instead of these, a single radiation shielding door having the functions of both the first and second radiation shielding doors 118 and 120 together may also be provided. Further, the target T is not limited to beryllium and lithium (Li), tantalum (Ta), tungsten (W), or the like may also be used.

Further, in the above-described embodiment, a configuration is also made such that the target T, the accommodation section 102, and the converging section 104 can relatively move with respect to each other. However, there is also a case where they do not relatively move with respect to each other, and in short, it is acceptable if the treatment table 32 is relatively moved with respect to the target T, the accommodation section 102, and the converging section 104.

### Industrial Applicability

According to the invention, it becomes possible to enhance work efficiency.

## Claims

1. A neutron beam irradiation system (1) adapted to irradiate a to be irradiated body (34) with a neutron beam (N), the neutron beam irradiation system (1) comprising:
a neutron beam generating section (T) adapted to generate said neutron beam (N) by irradiation with a charged particle beam (P);
an accommodation section (102) that includes at least a moderator (108) and accommodates the neutron beam generating section (T);
a neutron beam converging section (104) adapted to converge the neutron beam (N) which is irradiated to the to be irradiated body (34) ;
a placement table (32) on which the to be irradiated body (34) is placed; **characterised by**
a movement means (114, 116, 117) adapted to move the placement table (32) relative to the neutron beam generating section (T), the accommodation section (102), and the neutron beam converging section (104) so as to approach and be separated from the neutron beam generating section (T), the accommodation section (102), and the neutron beam converging section (104); and by
a first radiation shielding door (118) disposed between the neutron beam generating section (T) and the placement table (32) in a state where the neutron beam generating section (T) and the placement table (32) are separated from each other with respect to a positional relationship at the time of generation of the neutron beam (N).

2. The neutron beam irradiation system (1) according to Claim 1, wherein the movement means (114, 116, 117) is further adapted to move the neutron beam converging section (104) relative to the neutron beam generating section (T) so as to approach and be separated from the neutron beam generating section (T).

3. The neutron beam irradiation system (1) according to Claim 2, further comprising: a second radiation shielding door (120) that is disposed between the neutron beam generating section (T) and the neutron beam converging section (104) in a state where the neutron beam generating section (T) and the neutron beam converging section (104) are separated from each other with respect to a positional relationship at the time of the generation of the neutron beam (N).

4. The neutron beam irradiation system (1) according to Claim 1, wherein the movement means (114, 116, 117) includes a rail (114).

5. The neutron beam irradiation system (1) according to Claim 1, further comprising: a radiation shielding wall (112) that accommodates the accommodation section (102) such that said accommodation section (102) is embedded therein.

6. The neutron beam irradiation system (1) according to Claim 1, wherein the movement means (114, 116, 117) is further adapted to move the accommodation section (102) relative to the neutron beam generating section (T) so as to approach and be separated from the neutron beam generating section (T).

7. The neutron beam irradiation system (1) according to Claim 6, wherein the neutron beam generating section (T) is fixed to an installation floor (F), and
the movement means (114, 116, 117) is further adapted to move the accommodation section (102) relative to the neutron beam generating section (T) by moving the accommodation section (102) with respect to the installation floor (F).

## Patentansprüche

1. Neutronenstrahlbestrahlungssystem (1), das ausgelegt ist, um einen zu bestrahlenden Körper (34) mit einem Neutronenstrahl (N) zu bestrahlen, wobei das Neutronenbestrahlungssystem (1) Folgendes aufweist:
einen Neutronenstrahlerzeugungsabschnitt (T), der ausgelegt ist, um den Neutronenstrahl (N) durch Bestrahlung mit einem Strahl (P) geladener Teilchen zu erzeugen;
einen Unterbringungsabschnitt (102), der zumindest einen Moderator (108) aufweist, und den Neutronenstrahlerzeugungsabschnitt (T) unterbringt;
einen Neutronenstrahlkonvergenzabschnitt (104), der ausgelegt ist, um den Neutronenstrahl (N) zu konvergieren, der zu dem zu bestrahlenden Körper (34) ausgestrahlt wird;
einen Positionierungstisch (32), auf dem der zu bestrahlende Körper (34) angeordnet wird; **gekennzeichnet durch**
ein Bewegungsmittel (114, 116, 117), das ausgelegt ist, um den Positionierungstisch (32) relativ zu dem Neutronenstrahlerzeugungsabschnitt (T), dem Unterbringungsabschnitt (102) und dem Neutronenstrahlkonvergenzabschnitt (104) zu bewegen, um sich dem Neutronenstrahlerzeugungsabschnitt (T), dem Unterbringungsabschnitt (102) und dem Neutronenstrahlkonvergenzabschnitt (104) anzunähern und von diesen getrennt zu werden; und durch eine Strahlenschutztür (118), die zwischen dem Neutronenstrahlerzeugungsabschnitt (T) und dem Positionierungstisch (32) in einem Zustand angeordnet ist, in dem der Neutronenstrahlerzeugungsabschnitt (T) und der Positionierungstisch (32) voneinander in Bezug auf eine Positionsbeziehung zu dem Zeitpunkt der Erzeugung des Neutronenstrahls (N) getrennt sind.

2. Neutronenstrahlbestrahlungssystem (1) gemäß Anspruch 1, wobei das Bewegungsmittel (114, 116, 117) ferner ausgelegt ist, um den Neutronenstrahlkonvergenzabschnitt (104) relativ zu dem Neutronenstrahlerzeugungsabschnitt (T) zu bewegen, um sich dem Neutronenstrahlerzeugungsabschnitt (T) anzunähern und von diesem getrennt zu werden.

3. Neutronenstrahlbestrahlungssystem (1) gemäß Anspruch 2, der ferner Folgendes aufweist:
eine zweite Bestrahlungsabschirmungstür (120), die zwischen dem Neutronenstrahlerzeugungsabschnitt (T) und dem Neutronenstrahlkonvergenzabschnitt (104) in einem Zustand angeordnet ist, in dem der Neutronenstrahlerzeugungsabschnitt (T) und der Neutronenstrahlkonvergenzabschnitt (104) von einander in Bezug auf eine Positionsbeziehung zum Zeitpunkt der Erzeugung des Neutronenstrahls (N) getrennt werden.

4. Neutronenstrahlbestrahlungssystem (1) gemäß Anspruch 1, wobei das Bewegungsmittel (114, 116, 117) eine Schiene (114) aufweist.

5. Neutronenstrahlbestrahlungssystem (1) gemäß Anspruch 1, das ferner Folgendes aufweist:
eine Bestrahlungsabschirmungswand (112), die den Unterbringungsabschnitt (102) so unterbringt, dass der Unterbringungsabschnitt (102) darin eingebettet ist.

6. Neutronenstrahlbestrahlungssystem (1) gemäß Anspruch 1, wobei das Bewegungsmittel (114, 116, 117) ferner ausgelegt ist, um den Unterbringungsabschnitt (102) relativ zu dem Neutronenstrahlerzeugungsabschnitt (T) so zu bewegen, dass er sich dem Neutronenstrahlerzeugungsabschnitt (T) annähert und von diesem getrennt wird.

7. Neutronenstrahlbestrahlungssystem (1) gemäß Anspruch 6, wobei der Neutronenstrahlerzeugungsabschnitt (T) an einem Installationsboden (F) befestigt ist, und
das Bewegungsmittel (114, 116, 117) ferner ausgelegt ist, um den Unterbringungsabschnitt (102) relativ zu dem Neutronenstrahlerzeugungsabschnitt (T) durch Bewegen des Unterbringungsabschnitts (102) in Bezug auf den Installationsboden (F) zu bewegen.

## Revendications

1. Système d'irradiation par faisceau de neutrons (1) adapté à irradier un corps à irradier (34) avec un faisceau de neutrons (N), le système d'irradiation par faisceau de neutrons (1) comprenant :
une section de génération de faisceau de neutrons (T) adaptée à générer ledit faisceau de neutrons (N) par irradiation avec un faisceau de particules chargées (P) ;
une section de logement (102) qui comprend au moins un modérateur (108) et qui accueille la section de génération de faisceau de neutrons (T) ;
une section de convergence de faisceau de neutrons (104) adaptée à faire converger le faisceau de neutrons (N) qui est irradié vers le corps à irradier (34) ;
une table de placement (32) sur laquelle est placé le corps à irradier (34) ;
**caractérisé en ce que** des moyens de déplacement (114, 116, 117) adaptés à déplacer la table de placement (32) par rapport à la section de génération de faisceau de neutrons (T), la section de logement (102), et la section de convergence de faisceau de neutrons (104) de façon à s'approcher et à s'éloigner de la section de génération de faisceau de neutrons (T), de la section de logement (102), et de la section de convergence de faisceau de neutrons (104) ; et **en ce que**
une première porte de protection contre les rayonnements (118) placée entre la section de génération de faisceau de neutrons (T) et la table de placement (32) dans un état dans lequel la section de génération de faisceau de neutrons (T) et la table de placement (32) sont séparées l'une de l'autre en fonction d'une relation de position à l'instant de la génération du faisceau de neutrons (N).

2. Système d'irradiation par faisceau de neutrons (1) selon la revendication 1, dans lequel les moyens de déplacement (114, 116, 117) sont en outre adaptés à déplacer la section de convergence de faisceau de neutrons (104) par rapport à la section de génération de faisceau de neutrons (T) de façon à se rapprocher et à s'éloigner de la section de génération de faisceau de neutrons (T) .

3. Système d'irradiation par faisceau de neutrons (1) selon la revendication 2, comprenant en outre : une deuxième porte de protection contre les rayonnements (120) qui est placée entre la section de génération de faisceau de neutrons (T) et la section de convergence de faisceau de neutrons (104) dans un état dans lequel la section de génération de faisceau de neutrons (T) et la section de convergence de faisceau de neutrons (104) sont séparées l'une de l'autre en fonction d'une relation de position à l'instant de la génération du faisceau de neutrons (N).

4. Système d'irradiation par faisceau de neutrons (1) selon la revendication 1, dans lequel les moyens de déplacement (114, 116, 117) comprennent un rail (114).

5. Système d'irradiation par faisceau de neutrons (1) selon la revendication 1, comprenant en outre : une paroi de protection contre les rayonnements (112) qui reçoit la section de logement (102) de sorte que ladite section de logement (102) soit encapsulée dedans.

6. Système d'irradiation par faisceau de neutrons (1) selon la revendication 1, dans lequel les moyens de déplacement (114, 116, 117) sont en outre adaptés à déplacer la section de logement (102) par rapport à la section de génération de faisceau de neutrons (T) de façon à s'approcher et s'éloigner de la section de génération de faisceau de neutrons (T).

7. Système d'irradiation par faisceau de neutrons (1) selon la revendication 6, dans lequel la section de génération de faisceau de neutrons (T) est fixée sur un socle d'installation (F), et
les moyens de déplacement (114, 116, 117) sont en outre adaptés à déplacer la section de logement (102) par rapport à la section de génération de faisceau de neutrons (T) en déplaçant la section de logement (102) par rapport au socle d'installation (F).
